# EUROPEAN PATENT APPLICATION

(11) **EP 3 171 176 A1**
(43) Date of publication of application: **24.05.2017**
(21) Application number: 16164121.2
(22) Date of filing: 06.04.2016
(51) Int. Cl.: G01N 33/84, G01N 33/96, G01N 33/68

(54) **METHODS AND MEANS FOR ASSESSING THE QUALITY OF A BIOLOGICAL SAMPLE**

(30) Priority: 19.11.2015 EP 15195301
(71) Applicant: Luxembourg Institute of Health, 1526 Luxembourg (LU)
(72) Inventor: BETSOU, Fay, 1750 Luxembourg (LU); TREZZI, Jean-Pierre, L-4814 Rodange (LU); HILLER, Karsten, D-66663 Merzig (DE)
(74) Representative: Lavoix

(57) **Abstract**

The present invention concerns methods and means for assessing the quality of a biological sample. Specifically, the present invention relates to a method for assessing the quality of a biological sample comprising the steps of measuring the concentration of lactate and of ascorbate of said biological sample.

## Description

The present invention concerns methods and means for assessing the quality of a biological sample. Specifically, the present invention relates to a method for assessing the quality of a biological sample comprising the steps of measuring the concentration of lactate and of ascorbate of said biological sample.

Analytical and diagnostics methods are influenced by preanalytical variables that interfere with the results and renders interpretation uncertain. Controlling preanalytical variables is a challenging and complex issue because the influence of the quality of a biological sample on the molecular data obtained from its analysis depends not only on the class of biomolecules analyzed but also on the type of analytical method. Two main approaches have been developed to address this issue. The first consists in the optimization of the quality of the biological sample to minimize and/or control preanalytical bias. The second approach is to apply tests to assess the global biomolecular integrity status of the biological sample. In this aim, various standards for quality assurance and quality control for biobanking exist and several quality biomarkers have been developed. In order to assess the quality of biological sample, at present, biochemical standard parameters, such as nucleic acid content and integrity, presence of coagulation activity, or cellular composition, cell integrity and number of cells in the sample are determined. The evaluation of such standard parameters, however, will not be suitable for a more sensitive quality assessment, for example for assessment of suitability for downstream metabolome analysis. Moreover there are only a few appropriate quality control tools that are predictive of downstream method feasibility and reliability, or are diagnostic of upstream sample processing steps.

Ascorbate and lactate have been reported as quality biomarkers in certain cases. Ascorbate has been cited as a potential quality biomarker in serum and plasma, its concentration diminishing with conservation and in particular at room temperature (Betsou et al. The J Mol Diag, 2013 15(1)). The concentration of lactate is known to increase in serum when the temperature increases and when the pre-centrifugation time increases (Fliniaux et al., J Biomol NMR, 2011 51:457-465). Regrettably in this article preanalytical variability was negligible compared to inter-individual variation, limiting the use of quality biomarkers such as lactate.

The inventors, by their work conducted at the Integrated Biobank of Luxembourg and at the Luxembourg Center for Systems Biomedicine, have demonstrated that the ratio of the concentration of ascorbate on the concentration of lactate can be used as a quality biomarker with a good sensitivity and specificity.

The present invention relates to a method for assessing the quality of a biological sample comprising:
(a) measuring the concentration of lactate and of ascorbate of said biological sample,
(b) calculating the value of the ratio of the concentration of ascorbate on the concentration of lactate,
(c) deducing from said ratio value the quality of the biological sample.

One advantage of this method is that the sensitivity is improved by the use of this particular ratio.

In a preferred embodiment, the method according to the invention, is performed ex *vivo* i.e. not performed on human or animal body and is preferably assisted by automation.

The term "assessing" as used herein refers to distinguishing between a good or poor quality and in particular between a platinum quality and a lower quality. A biological sample of platinum quality can be used for analytical studies or diagnostic methods such as lipidomics, transcriptomics, metabolomics and/or peptidomics in particular because preanalytical variables are deemed to be negligible. A biological sample of lower quality will be considered as not suitable for application such as lipidomics, transcriptomics, metabolomics and/or peptidomics because preanalytical variables may impact the results and thus lead to important variations, erratic effects and/or irreproducible results. A biological sample of lower quality can be used for analytical studies or diagnostic methods such as proteomics, immunological and/or biochemical analyses. A sample of lower quality can result from degradation of components and/or chemical alterations and may be due to adverse effects of preanalytical factors such as prolonged processing, hemolysis, microclotting, cellular contamination, improper storage conditions and/ or improper freezing such as slow freezing. In a preferred embodiment, the invention relates to a method for assessing the quality of a biological sample with respect to preanalytical conditions.

Assessing the quality of biological sample is helpful for an efficient metabolomic or peptidomic diagnosis of diseases or medical conditions where proper sample quality is decisive for a reliable diagnosis.

The term "biological sample" as used herein refers to sample comprising biological materials. Preferably the biological sample is a sample of body fluids such as cerebrospinal fluid, blood, saliva or urine. In a particular embodiment the biological sample is extracted from blood. In a preferred embodiment the biological sample is plasma or serum, most preferably the biological sample is plasma. The biological samples can be derived from a subject. Techniques for obtaining the aforementioned different type of biological samples are well known in the art; for example, blood samples may be obtained by blood taking while tissue or organ sample are to be obtained e.g. by biopsy. In a preferred embodiment, a biological sample according to the invention has not been conserved in citrate and/or heparin and/or meta-phosphoric acid. In a particularly preferred embodiment, the biological sample is taken and conserved in EDTA.

A subject as used herein relates to animals and preferably to mammals. More preferably the subject is a rodent and most preferably a mouse or a rate. In another embodiment of the invention the subject is a primate and most preferably a human. The subject can be suspected to suffer from a disease or medical condition, or not, or be at risk for developing a disease or medical condition, or not. In a preferred embodiment, the subject is exempt of extremely high lactate inducing conditions such as intensive exercise, ethanolaemia, acute sepsis, cardiac arrest or trauma and/or abnormal ascorbic acid concentration conditions such as due to the ascorbic acid supplementation, stroke, acute pancreatitis or hyperoxalaemia. In a preferred embodiment, the biological sample is conserved in EDTA and the ascorbate concentration is inferior to 15 µM and the lactate concentration is inferior to 10mM.

For example a plasma sample of platinum quality refers to a sample kept in ice for less than 3 hours before centrifugation and preferably less than 1 hour and then frozen at -80°C or to a sample kept at room temperature (i.e. between 18°C-26°C) for less than 3 hours before centrifugation; and a sample of lower quality refers to a sample stored for 3 hours or longer before centrifugation.

The term "ascorbate" as used herein refers to 2-oxo-L-threo-hexono-1,4-lactone-2,3-enediol, it includes Vitamin C also named L-ascorbic acid and its salts, and ascorbate (the anion of ascorbic acid).

The term "lactate" as used herein refers to 2-Hydroxypropanoic acid commonly named lactic acid and its salts.

The term "metabolomics" as used herein refers to the analysis of metabolite profiles of biological samples, especially in response to various stressors, at various developmental stages, or in various parts of the body. The metabolome represents the collection of all metabolites in a biological cell, tissue, organ or organism, which are the end products of cellular processes. Metabolic profiling can give an instantaneous snapshot of a cell and is the molecular profiling that evolves in the most dynamic manner.

The term "peptidomics" as used herein refers to the analysis of peptides profiles of biological samples, especially in response to various stressors, at various developmental stages, or in various parts of the body. Peptidic profiling gives another aspect of cellular physiology.

The measurement of the concentrations of lactate and ascorbate according to the invention may be realized by any method known by the skilled in the art. In particular chromatography, mass spectrometry, proton nuclear magnetic resonance spectroscopy, enzymatic assays, spectrophotometry, and/or spectrofluorometry can be used. In a particular embodiment the concentration of ascorbate is measured by fluorimetry or colorimetry. In a particular embodiment the concentration of lactate is measured by chromatography. In an embodiment concentrations of lactate and ascobate are measured by gas chromatography-mass spectrometry (GC-MS). In a preferred embodiment the measurement of ascorbate does not include the quantity of dehydroascorbic acid of the biological sample.

In a particular embodiment, step (c) is performed by comparison of the obtained ratio with a reference value. In an embodiment the reference value is determined by the mean value of the ratio of the concentration of ascorbate on the concentration of lactate of known platinum quality samples. In another embodiment the reference value is determined by the mean value of the ratio of the concentration of ascorbate on the concentration of lactate of known lower quality samples. In another embodiment the reference value is the optimal cut-off value determined by ROC analysis. In accordance a reference value is determined by a plurality of sample preferably more than 2, 10 , 50 or 100 samples.

The term "comparison" refers to determining whether the value of the ratio is essentially identical to a reference value or differs therefrom. Preferably, a value for the ratio is deemed to differ from a reference value if the observed difference is statistically significant, which can be determined by statistical techniques. If the difference is not statistically different, the ratio value and the reference value are essentially identical. Based on the comparison, the quality of a sample can be assessed, i.e. it can be assessed whether the samples is of platinum quality, or not.

The skilled in the art is able to deduce from the ratio value the quality of the biological sample according to the chosen reference. Typically the biological sample is considered of platinum quality when the calculated ratio is equivalent to the reference value and the biological sample is considered of lower quality when the calculated ratio is inferior to the reference value. For example if the reference value chosen is the mean ratio of known platinum quality samples, sample having a ratio higher or similar to the reference value will be considered of platinum quality, sample having a ratio lower to the reference value but not significantly will be considered of uncertain quality and sample having a ratio significantly lower to the reference value will be considered of lower quality.

The comparison is, preferably assisted by automation. For example, a suitable computer program comprising algorithms for the comparison of two different data sets may be used. Other algorithms such as a learning algorithm may be used to improve the reference value or the threshold. Such programs and algorithms are well known in the art.

Notwithstanding the above, a comparison can also be carried out manually.

As will be understood by those skilled in the art, such an assessment, although preferred to be, may usually not be correct for 100% of the investigated samples. The term, however, requires that a statistically significant portion of samples can be correctly assessed. Whether a portion is statistically significant can be determined by the person skilled in the art using various well-known statistic evaluation tools, e.g., determination of the confidence intervals, p- value determination, Student's t-test, Mann-Whitney test, etc. Preferred confidence intervals are at least 50%, at least 60 %, at least 70 %, at least 80 %, at least 90 % or at least 95 %. The p-value are, preferably 0.2, 0.1 or 0.05.

In an embodiment the method of the present invention comprises one or more additional steps such as a pretreatment or a deduction of the suitability of the biological sample for specific applications.

The term "pretreatment" refers to treatments required, for example, to release, separate compounds, sterilize or remove contaminants. Such treatments take place before step (a) and include centrifugation, extraction, protein precipitation, freeze-drying, drying, filtration, fractionation, vaporization, and enrichment of compounds. Pretreatment also comprise treatments carried out to render the sample suitable for the chosen compound analysis. These methods are not further described herein as they are well-known to the person skilled in the art. Another kind of pretreatment may be storage of the samples under suitable storage conditions. Storage conditions include storage temperature, pressure, humidity, time as well as the treatment of the stored sample with preserving agents. Suitable and necessary pretreatments also depends on the means used for carrying the method of the invention and are well known to the person skilled in the art. Pretreated samples are also comprised by the term biological sample as used in accordance with the present invention.

In a particular embodiment the method according to the invention is used to assess at least one biological sample from a collection of samples having been processed in similar ways, or a batch, and thus allows deducing the quality of the whole collection or batch.

The present invention also relates to the use of a system for quantifying ascorbate and lactate for assessing the quality of a biological sample based on the calculation of the ratio of the concentration of ascorbate on the concentration of lactate. Those system for quantifying ascorbate and lactate includes chromatograph, mass spectrometer, proton nuclear magnetic resonance spectroscope, spectrophotometer and/or spectrofluorometer.

The present invention also relates to the use of enzymes for quantifying ascorbate and lactate for assessing the quality of a biological sample based on the calculation of the ratio of the concentration of ascorbate on the concentration of lactate. In a preferred embodiment those enzymes react with ascorbate or lactate and produce a detectable compound. For example those enzymes for quantifying ascorbate and lactate include lactate dehydrogenase and ascorbate oxidase.

The present invention also relates to the use of a combination of at least a first kit comprising a system for quantifying ascorbate, and a second kit comprising a system for quantifying lactate, for assessing the quality of a biological sample based on the calculation of the ratio of the concentration of ascorbate on the concentration of lactate.

The present invention also relates to the use of a combination of at least a first kit comprising an enzyme for quantifying ascorbate, and a second kit comprising an enzyme for quantifying lactate, for assessing the quality of a biological sample based on the calculation of the ratio of the concentration of ascorbate on the concentration of lactate.

The invention will be illustrated by the following figures and examples which are not intended to restrict or limit the scope of the invention.

### FIGURES

Figure 1 shows the influence of preanalytical conditions on the ascorbate/lactate (Asc/Lac) ratio after short term pre-centrifugation delays and when ascorbate and lactate are measured by GC/MS.
Figure 2 shows the influence of preanalytical conditions on the serine/tyrosine ratio after short term pre-centrifugation delays and when serine and tyrosine are measured by GC/MS.
Figure 3 shows the influence of preanalytical conditions on the ascorbate/lactate (Asc/Lac) ratio after longer pre-centrifugation delays and when ascorbate and lactate are measured by spectrophoto/fluorometric methods.

### EXAMPLES

### Example 1:

Plasma samples from 3 healthy donors were subdivided in 6 aliquots. Each of them underwent different preanalytical conditions i.e.:
- 10 minutes at 4°C before centrifugation and freezing,
- 30 minutes at 4°C before centrifugation and freezing,
- 60 minutes at 4°C before centrifugation and freezing,
- 10 minutes at 25°C before centrifugation and freezing,
- 30 minutes at 25°C before centrifugation and freezing, and
- 60 minutes at 25°C before centrifugation and freezing.

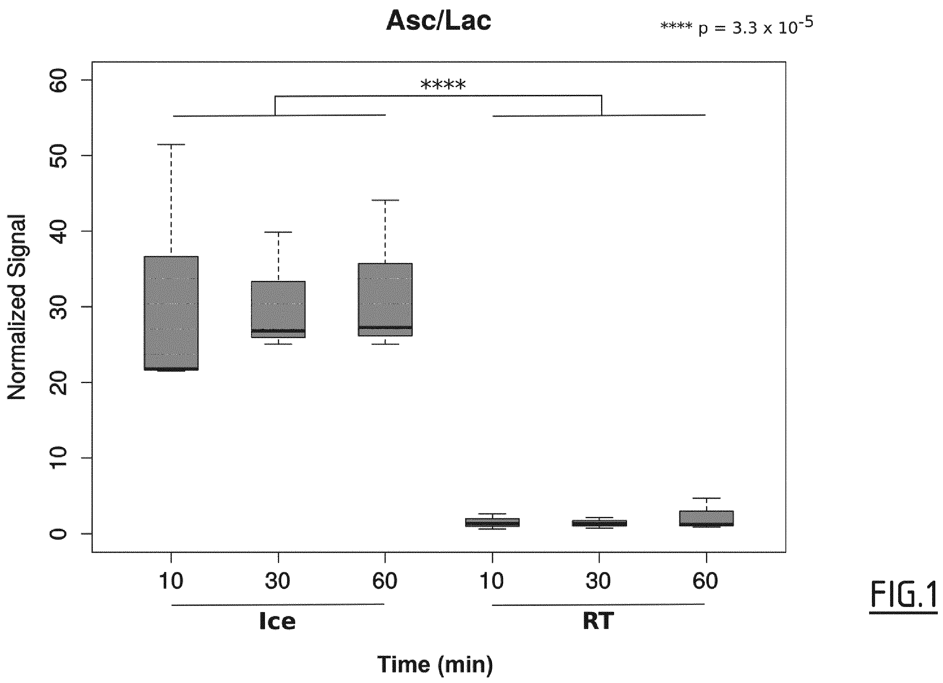

The amount of lactate and ascorbate of each sample were measured by GC-MS after short pre-centrifugation delays (10-60 min), Figure 1 displays a box plot of the obtained ascorbate/lactate (Asc/Lac) ratio of each aliquot.

The value of the ratio of samples conserved in ice before freezing is significantly superior to the value of the ratio of samples conserved at room temperature (p-value<0.0001).

### Example 2:

Plasma samples from 3 healthy donors were subdivided in 6 aliquots. Each of them underwent the same preanalytical conditions as in example 1.

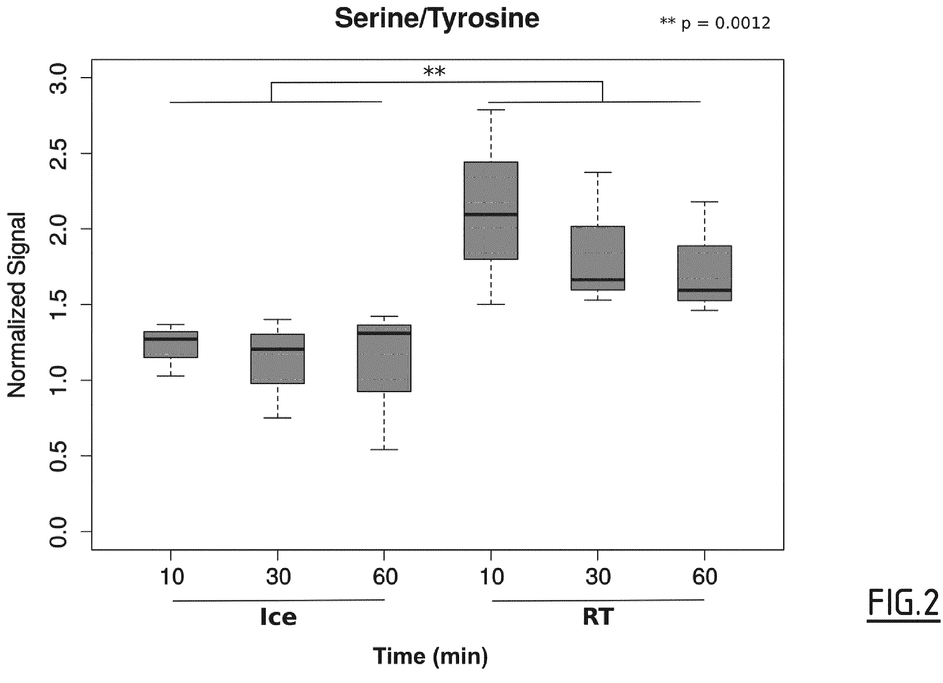

The amount of serine and tyrosine of each sample were measured by GC-MS, Figure 2 displays a box plot of the obtained serine/tyrosine ratio of each aliquot.

The value of the ratio of samples conserved in ice before freezing is significantly inferior to the value of the ratio of samples conserved at room temperature but to a lesser extent than from the ascorbate/lactate ratio. The ratio serine/tyrosine is thus a biomarker with less sensitivity than ascorbate/lactate ratio.

### Example 3:

Plasma samples from 8 healthy donors were subdivided in 6 aliquots. Each of them underwent the different preanalytical conditions:
- 30 minutes at 4°C before centrifugation and freezing,
- 3 hours at 4°C before centrifugation and freezing,
- 23 hours at 4°C before centrifugation and freezing,
- 30 minutes at 25°C before centrifugation and freezing,
- 3 hours at 25°C before centrifugation and freezing, and
- 23 hours at 25°C before centrifugation and freezing.

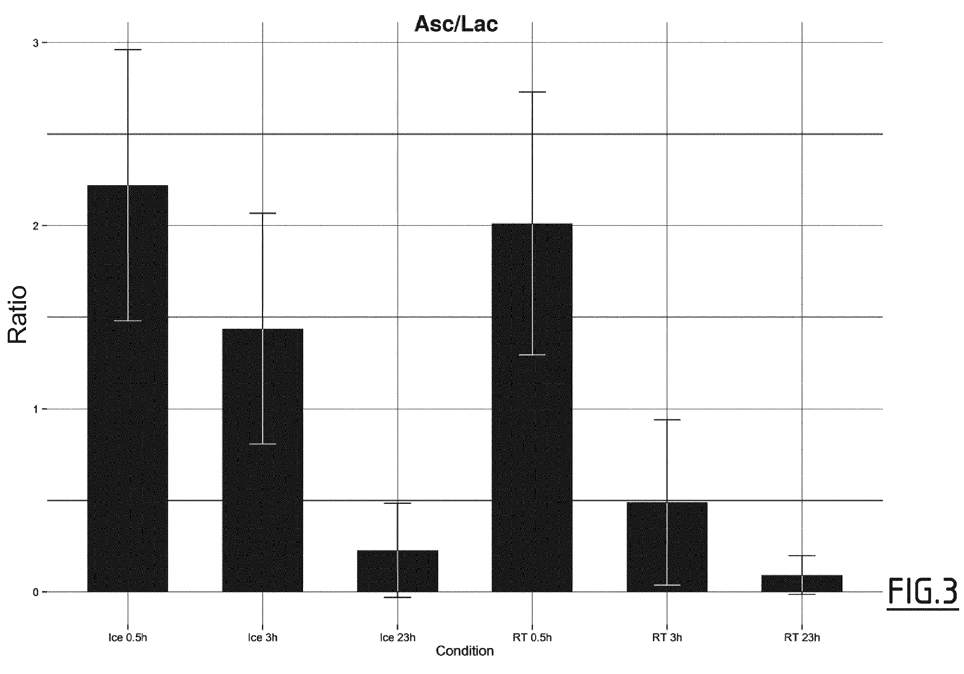

The amount of lactate and ascorbate of each sample were measured by spectrophoto/fluorometric methods after 30 minutes, 3 hours and 23 hours pre-centrifugation delays, Figure 3 displays a histogram of the obtained ascorbate/lactate (Asc/Lac) ratio of each aliquot.
We observed significant differences in the ascorbate/lactate ratio between blood aliquots kept in ice for 3 hours or at room temperature for 30 min on the one hand, and aliquots kept in ice for 23 hours or at room temperature for 3 hours on the other hand.

## Claims

1. A method for assessing the quality of a biological sample comprising:
(a) measuring the concentration of lactate and of ascorbate of said biological sample,
(b) calculating the value of the ratio of the concentration of ascorbate on the concentration of lactate,
(c) deducing from the said ratio value the quality of the biological sample.

2. The method according to claim 1, wherein the said ratio is compared with a reference value.

3. The method according to claim 2, wherein the said biological sample is considered of platinum quality when said ratio is superior to the reference value and said biological sample is considered of lower quality when said ratio is inferior to the reference value.

4. The method according to any one of claims 1 to 3, further comprising (d) deducing from the quality of said biological sample its suitability for applications in metabolomics and/or peptidomics.

5. The method according to claim 1 or 4, wherein step (a) is performed by chromatography and/or mass spectrometry and/or proton nuclear magnetic resonance spectroscopy, and/or spectrophotometry, and/or spectrofluorometry.

6. The method according to any one of claims 1 to 5, wherein the biological sample is pretreated before step (a) by at least one method chosen in the group consisting of centrifugation, extraction, protein precipitation, freeze-drying, drying, filtration, fractionation, vaporization and enrichment of compounds.

7. The method according to any one of claims 1 to 6, wherein ascorbate is measured by fluorimetry.

8. The method according to any one of claim 1 to 7, wherein the biological sample is plasma.

9. The method according to any one of claim 1 to 7, wherein the biological sample is taken and conserved in EDTA.

10. Use of means for quantifying ascorbate and lactate for assessing the quality of a biological sample based on the calculation of the ratio of the concentration of ascorbate on the concentration of lactate.

11. Use of a combination of at least a first kit comprising means for quantifying ascorbate, and a second kit comprising means for quantifying lactate, for assessing the quality of a biological sample based on the calculation of the ratio of the concentration of ascorbate on the concentration of lactate.

## Amended claims

### Amended claims in accordance with Rule 137(2) EPC.

1. A method for assessing the quality of a biological sample comprising:
(a) measuring the concentration of lactate and of ascorbate of said biological sample,
(b) calculating the value of the ratio of the concentration of ascorbate on the concentration of lactate,
(c) deducing from the said ratio value the quality of the biological sample.

2. The method according to claim 1, wherein the said ratio is compared with a reference value.

3. The method according to claim 2, wherein the said biological sample is considered of platinum quality when said ratio is superior to the reference value and said biological sample is considered of lower quality when said ratio is inferior to the reference value.

4. The method according to any one of claims 1 to 3, further comprising (d) deducing from the quality of said biological sample its suitability for applications in metabolomics and/or peptidomics.

5. The method according to claim 1 or 4, wherein step (a) is performed by chromatography and/or mass spectrometry and/or proton nuclear magnetic resonance spectroscopy, and/or spectrophotometry, and/or spectrofluorometry.

6. The method according to any one of claims 1 to 5, wherein the biological sample is pretreated before step (a) by at least one method chosen in the group consisting of centrifugation, extraction, protein precipitation, freeze-drying, drying, filtration, fractionation, vaporization and enrichment of compounds.

7. The method according to any one of claims 1 to 6, wherein ascorbate is measured by fluorimetry.

8. The method according to any one of claim 1 to 7, wherein the biological sample is plasma.

9. The method according to any one of claim 1 to 7, wherein the biological sample is taken and conserved in EDTA.

10. Use of systems and/or enzymes for quantifying ascorbate and lactate for assessing the quality of a biological sample based on the calculation of the ratio of the concentration of ascorbate on the concentration of lactate.

11. Use of a combination of at least a first kit comprising systems and/or enzymes for quantifying ascorbate, and a second kit comprising systems and/or enzymes for quantifying lactate, for assessing the quality of a biological sample based on the calculation of the ratio of the concentration of ascorbate on the concentration of lactate.
